# EUROPEAN PATENT SPECIFICATION

(11) **EP 4 409 292 B1**
(45) Date of publication and mention of the grant of the patent: **05.11.2025**
(21) Application number: 21847786.7
(22) Date of filing: 30.12.2021
(51) Int. Cl.: G01N 33/58, G01N 33/543, G01N 33/546, G01N 33/68, G01N 33/542, B82Y 15/00

(54) **IN VITRO POINT-OF-CARE ASSAY METHOD/PROCESS USING A COMBINATION OF BIOACTIVATED NON-MAGNETIC AND MAGNETIC QUANTUM DOTS FOR RAPID IMMUNO-OPTOMAGNETIC DETECTION OF SERUM BIOMARKERS IN FREE SOLUTION**
IN-VITRO-TESTVERFAHREN/-PROZESS FÜR DEN EINSATZ AM VERSORGUNGSORT MIT EINER KOMBINATION AUS BIOAKTIVIERTEN NICHTMAGNETISCHEN UND MAGNETISCHEN QUANTENPUNKTEN
PROCÉDÉ/PROCESSUS DE DOSAGE IN VITRO DE POINT D'INTERVENTION UTILISANT UNE COMBINAISON DE POINTS QUANTIQUES BIOACTIVÉS NON MAGNÉTIQUES ET MAGNÉTIQUES POUR LA DÉTECTION IMMUNO-OPTOMAGNÉTIQUE RAPIDE DE BIOMARQUEURS SÉRIQUES EN SOLUTION LIBRE

(43) Date of publication of application: 07.08.2024
(73) Proprietor: Sabanci Universitesi Nanoteknoloji Arastirma Ve Uygulama Merkezi, Tuzla/Istanbul (TR)
(72) Inventor: KOLKAR MOHAMMED, Javed Hussain Niazi, 34956 Tuzla/ Istanbul (TR); QURESHI, Anjum, 34956 Tuzla/ Istanbul (TR)
(74) Representative: Yalçiner Patent and Consulting Ltd.
(86) International application number: PCT/TR2021/051627
(87) International publication number: WO 2023/128904

(56) References cited:
- WO-A1-2021/167542
- HO YI-PING ET AL: "Multiplexed Hybridization Detection with Multicolor Colocalization of Quantum Dot Nanoprobes", NANO LETTERS, vol. 5, no. 9, 2 August 2005 (2005-08-02), US, pages 1693 - 1697, XP055847426, ISSN: 1530-6984, Retrieved from the Internet <URL:https://pubs.acs.org/doi/pdf/10.1021/nl050888v> DOI: 10.1021/nl050888v
- LIU JIANBO ET AL: "Solid-phase single molecule biosensing using dual-color colocalization of fluorescent quantum dot nanoprobes", NANOSCALE, vol. 5, no. 22, 16 September 2013 (2013-09-16), United Kingdom, pages 11257, XP055946165, ISSN: 2040-3364, Retrieved from the Internet <URL:https://pubs.rsc.org/en/content/articlepdf/2013/nr/c3nr03291d> DOI: 10.1039/c3nr03291d
- JAROCKYTE GRETA ET AL: "Multiplexed Nanobiosensors: Current Trends in Early Diagnostics", SENSORS, vol. 20, no. 23, 2 December 2020 (2020-12-02), pages 6890, XP055945645, Retrieved from the Internet <URL:https://www.ncbi.nlm.nih.gov/pmc/articles/PMC7729484/pdf/sensors-20-06890.pdf> DOI: 10.3390/s20236890
- HEO JUN HYUK ET AL: "A significant enhancement of color transition from an on-off type achromatic colorimetric nanosensor for highly sensitive multi-analyte detection with the naked eye", NANOSCALE, vol. 8, no. 43, 5 October 2016 (2016-10-05), United Kingdom, pages 18341 - 18351, XP055777276, ISSN: 2040-3364, Retrieved from the Internet <URL:https://pubs.rsc.org/en/content/articlepdf/2016/nr/c6nr05919h> DOI: 10.1039/C6NR05919H
- JIANG WENXIAO ET AL: "A Dual-Color Quantum Dots Encoded Frit-Based Immunoassay for Visual Detection of Aflatoxin M 1 and Pirlimycin Residues in Milk", JOURNAL OF AGRICULTURAL AND FOOD CHEMISTRY, vol. 65, no. 8, 13 February 2017 (2017-02-13), US, pages 1822 - 1828, XP055945423, ISSN: 0021-8561, DOI: 10.1021/acs.jafc.6b05337
- PI JIANGLI ET AL: "A sandwich immunoassay for detection of A[beta]1-42based on quantum dots", TALANTA, vol. 146, 14 August 2015 (2015-08-14), pages 10 - 15, XP029349491, ISSN: 0039-9140, DOI: 10.1016/J.TALANTA.2015.08.022
- PERKINELMER: "AlphaLISA Amyloid beta 1-40 HS Detection Kit - AL373C/F", 31 December 2012 (2012-12-31), XP055945934, Retrieved from the Internet <URL:https://resources.perkinelmer.com/lab-solutions/resources/docs/TDS_AlphaLISA_Ab40_HS_AL373.pdf?_ga=2.126076429.1249286016.1658755325-617172286.1658755325>

## Description

### Technical Field of the Invention

The present invention relates to a low-cost, rapid, real-time and simple immuno-optomagnetic point-of-care (PoC) assay. More particularly, the present invention relates to a method for multiplexed detection of a panel of four biomarker proteins in serum for the diagnosis of Alzheimer's disease and it does not require a glass chip platform to generate the signal.

### Known State of the At (Prior Art)

Alzheimer's disease (AD) is an incurable disorder characterized by progressive memory loss, mild cognitive impairment (MCI) to profound cognitive failure. In 2019, World Health Organization (WHO) has recognized AD and dementia as a seventh leading cause of death (worldwide 2.7%) among top 10 leading causes of death in the World. About 65% of deaths from Alzheimer's disease and other forms of dementia are women (https://www.who.int/news-room/factsheets/detail/the-top-10-causes-of-death).

Longer life-expectancies have led to an increased number of neurodegenerative diseases globally [1]. The number of people with Alzheimer's disease or dementia in Turkey alone is reported to be 331,512 in 2012, which represents 0.44% of total population at the time. This number has now increased to 528,547 cases which is ~ 0.65% of Turkish population according to 2019 reports in the EU's Alzheimer Europe Yearbook [2]. In another survey limited to a total 3100 inhabitant population of Eskisehir city in Turkey showed that 51.1% of these inhabitant population were suffering from the most common type of disease was vascular dementia. The same study also found that the remaining 48.8% were found to be suffering from Alzheimer's disease (AD) and 0.1% were with other type of dementias [3, 4]. Turkey's Statistics Institute report suggests that the AD will rise to over 10% in elderly population over age 65 years by 2023 and continue to increase rapidly [5]. The exact reason for the prevalence and risk of dementia is unclear, however Turkey's rapid development and urbanization have been considered to have influence on this disease [6].

In general, much of AD cases have been found sporadic in nature, while there is increasing evidence from the population-based neurological and neuroimaging studies showing that mixed AD and vascular brain pathologies accounts for most of dementia cases in elderly people [7].

AD is linked to multiple risk factors including lifestyle, smoking, alcohol misuse, unhealthy diet that are the sources for a range of diseases that are ultimately linked to dementia.

Current understanding for interventions to prevent the AD or dementia is somewhat disappointing, because the multi-factorial and heterogeneous character of AD is only identified in its late-onset. Timing of AD diagnosis becomes critical especially when the pathology process begins 10-15 years before it shows its symptoms [8]. However, it is difficult to determine the exact time of onset of AD. Lately, there is a shift toward early diagnosis of AD which relies on early identification of significant episodic memory impairment and use of distinctive and reliable biomarkers of AD as revised diagnostic criteria [9].

Earlier standard measures for the diagnosis or clinical manifestations of AD has been through cognitive dysfunction and impairments of activities. It is followed by the neuroimaging using MRI allowing detailed examination of brain structure and hippocampal volume measurement or histopathological identification of amyloid plaques after postmortem [10].

All the existing traditional diagnosis methods fail to address the progression of the Alzheimer's disease even before it precedes or show symptoms in suspected individuals. There is an enormous gap in early diagnosis and monitoring progression of AD in a minimally non-invasive way that remains to be fulfilled.

There is a potential for identifying AD at the very early stages and its progression by a minimally-invasive, easily accessible blood serum/plasma biomarker measure for effective diagnosis, which required extensive research on utilizing a class of AD-biomarkers for PoC tools for early diagnosis. Therefore, detection of AD-biomarkers in blood serum/plasma require standardization before forming core elements of AD diagnostic criteria. Detection of biomarkers in blood serum/plasma, such as amyloid β40/β42, tau protein and BDNF are among the most promising AD diagnostic methods. However, these biomarkers occur in very low concentrations in blood serum/plasma which makes it extremely difficult to precisely and accurately detect such biomarkers.

Most current detection methods rely on a single biomarker for AD diagnosis, which is highly unreliable for accurate AD diagnosis [11]. Clinical standardization of AD diagnosis based on AD biomarker levels in serum/plasma or CSF is needed, which is currently unclear.

Limited attempts have been made thus far in detecting multiple AD biomarkers from a sample that has great potential in enhancing the accuracy of AD diagnosis [8]. Simultaneous measurement of multiple biomarkers present in a sample could provide a high degree of accuracy in diagnosis of AD.

In a few cases, ratio of a pair of biomarkers, such as CSF or blood plasma Aβ42:Aβ40 has been used as indicator for the disease due abnormal Aβ metabolism, and in other cases, increased T-tau and P-tau due to neuronal damage and accumulation of tau have also been used for diagnosis of AD [1]. All these detection methods required laborious proteomics, magnetic resonance or spectroscopic tools for diagnosis that are expensive and time-consuming.

There is ever increasing demand for more accessible and affordable healthcare for chronic as well as infectious diseases. Extension of traditional diagnostic approaches to PoC diagnostics is critically important for affordable, rapid and accurate diagnosis of chronic diseases, such as AD that currently presents significant challenges. PoC based diagnosis offer advantageous of speed, sensitivity, specificity, and compatibility of using them near the patient bedside. Recent developments in mobile technologies, nanotechnology, imaging systems and microfluidics have transformed the innovative solutions toward developing new, low cost and portable PoC diagnostics [12].

In the last five years, major advancement took place in nanotechnology exploiting the unique physico-chemical properties of nanoparticles, nanocrystals and other nanomaterials. These nano-sized materials are found to have many applications, including in biosensors or bioanalytical assays and their improvement. Nanomaterials have started to use in construction of biosensor based on classical antibodies (immunosensors) that remain the most common recognition elements in research and commercial affinity assays. Novel strategies for sensitive detection of biological responses in healthcare continue to be a priority for PoC applications. The current approaches require improvement toward developing novel multiplexed detection systems and nanomaterials-based research, exploiting the use of multimodal nanoparticles which will contribute to simpler and more sensitive bioanalysis suitable for PoC detection.

Previously, ELISA based methods have been proved to be most sensitive but rely on detection of a lone biomarker at a time, which is time consuming and highly expensive. Simultaneous measurement of multiple biomarkers present in a sample could provide a high degree of accuracy in diagnosis of AD. In a few cases, ratio of a pair of biomarkers, such as CSF or blood plasma Aβ42:Aβ40 has been used as indicator for the disease due to abnormal Aβ metabolism, and in other cases, increased T-tau and P-tau due to neuronal damage and accumulation of tau have also been used for diagnosis of AD.

All these existing detection methods required laborious proteomics or magnetic resonance (MR) or spectroscopic tools for diagnosis that are expensive and time-consuming. Many different commercial devices are available for the simultaneous detection of clinical parameters, including electrolytes or acute metabolites. For example, Abbott i-STAT system, Abaxis Piccolo Xpress or Novo StatSensor) or immunoassays such as AQT90 Radiometer and PATHFAST analyzer [13, 14]. There has been expensive and complicated assays performed for detecting varying levels of Aβ42, tau protein and BDNF biomarkers using traditional immunoassay or ELISA based detection, respectively [11], [15]. However, these systems are expensive or bulky bench-top analyzers only capable of detecting limited type of analytes in centralized clinical laboratories. Multiplexed PoC testing is a simultaneous on-site detection method for different analytes from a single specimen, especially at the point-of-care (PoC) settings, where an immediate decision on treatment needs to be made. Therefore, multiplexed detection by PoC system ensures the quality and performance of in vitro diagnostics, which will pave the way for novel health monitoring at home and add valuable information for personalized medicine. PoC testing decentralizes the traditional laboratory setting that greatly reduce the risk and increase interaction of laboratory personnel with patients and other healthcare team.

Our previous invention (PCT/TR2020/050115) relates to the immuno-optomagnetic Point-of-Care (PoC) assay and method for detection of hErbB2 (Her2) serum biomarker specific to breast cancer. This patent document describes a combination of following two major components for detection of a model biomarker (hErbB2 protein) in serum; (i) pre-activated PoC-chip platform that carried a Ab1 antibody on chip, and (ii) bioactivated magnetic quantum dots (MQDs-Ab2-x). The assay method originally designed to carry out on a PoC-chip platform in two sequential steps. First, MQDs-Ab2-x specifically captures protein biomarker analyte (hErbB2 protein) from the test serum samples to form "Complex-1". Second, this Complex-1 is then sandwiched on pre-activated PoC chip to form a sandwich Complex-2. The Complex-2 can later be visualized for the detection and quantification of analyte.

The above process related to our previous invention was although sensitive, but it is relatively tedious and require more cost for the whole assay to perform. Therefore, in the present invention, the assay method/process is re-designed to provide more simpler and cost-effective approach to in vitro PoC assay method/process that eliminates the requirement of a PoC-chip component and reducing the cost of the assay process/method. Also, speed of assay and real-time detection of diagnostic signal which is much simpler than the previously demonstrated assay/process in PCT/TR2020/050115.

Until now, there is no standard tool to diagnose MCI or AD before it precedes. Therefore, it is critically important to develop innovative method for a low-cost, equipment-free, accurate and early diagnosis of MCI or AD. Current understanding in relation to shaping future diagnosis of AD suggest that early diagnosis of AD can only be possible in a minimally invasive way through probing the bodily responses to the disease, such as screening for AD-specific biomarkers released in the body fluids.

There are a few known possibilities where the fractions of these biomarkers (cleaved oligomers) can be found in cerebral spinal fluid (CSF) or blood serum, or plasma. These oligomer fractions are diffusible proteins derived from amyloid-β (Aβ) and tau-protein or phosphorylated tau-protein (P-tau) that indicates AD-pathology and serve as signals for AD. Such proteins can be targeted for early diagnosis of MCI, AD or dementia. The current challenge is to develop an affordable, sensitive, equipment-free, and simple PoC assay and method for early AD diagnosis at a patient bedside or at homes.

### Brief Description of the Invention and its Aims

In the present invention, multi-colored CdSe/CdS/ZnS non-magnetic free/colloidal quantum dots (QDs) and magnetic quantum dots (MQDs) were "re-purposed" for developing a new equipment-free "solution phase PoC in vitro assay".

Following are the main differences between the present invention and our previous PCT/TR2020/050115 patent document:
(i) The main difference between the current invention and our previous invention of PCT/TR2020/050115 is that the current invention does not utilize PoC-chip made of glass substrate for detecting the signal.
(ii) Instead of PoC-chip, the present invention utilizes free/colloidal and non-magnetic QDs of multiple colors to generate the diagnostic signal.
(iii) The present invention is related to utilize a combination of bioactivated MQDs and non-magnetic QDs with multiple wavelength/colors to generate diagnostic signal against the biomarkers in serum. The signal is generated by means of transformation in primary colors/intensities of MQDs and non-magnetic QDs brought on by capturing the serum biomarkers. In other words, the detection of diagnostic signal originates from the color change after binding of an analyte with two different colored (wavelengths) of biofunctionalized MQDs (λ1) and bioactivated QDs (λ2) to give rise to a new color (λ1:λ2) of mixture.
(iv) The new PoC in vitro assay method designed for direct and visible signal detection in real-time is carried out in a small reaction microtube with a few tens of microliter volume that saves time and cost.
(v) The extended invention method/process involves multiplexed detection of a panel of four different Alzheimer's disease (AD) biomarkers as model protein analytes for a single disease type (AD), enabling accurate diagnosis.
(vi) A panel of four biomarker proteins tested in the present invention include two variants of amyloid β (Aβ) proteins, such as (a) Aβ1-40 and (b) Aβ1-42, along with two distinct biomarkers, such as (c) tau-protein and (d) Brain-Derived Neurotrophic Factor (BDNF) in human serum. These biomarkers are released from the brain of suspected patients into the body fluids that indicate the risk of age-related Dementia, Mild Cognitive Impairment and Alzheimer's disease in elderly individuals.
(vii) The dynamic range of detection established using the developed assay process/method with Aβ1-40 = 0.31-5 ng mL⁻¹, Aβ1-42 = 0.31~10 ng mL⁻¹, tau-protein = 0.07~1.25 ng mL⁻¹ and BDNF protein = 0.31 to above 10 ng mL⁻¹ that cover the window within the abnormal range and to diagnose disease risk.
(viii) The new assay method and protocol designed in the present invention is easy-to-use and cost-effective compared to the previous invention of PCT/TR2020/050115.
(ix) The visible signal is generated within 10~20 min after the sample addition, while it took at least 30 min in the previous invention.
(x) The signal generated is directly visible to a naked eye in real-time after exposing the tubes to a UV-torch, while in previous invention, an additional step as well as a glass chip platform was required that took extra cost for assay and additional assay time.
(xi) The assay in present invention does not require isolation of reaction components from the tube, which is another major difference to detect the signal compared to our previous invention, nor the newly designed assay required any sample preparation steps.
(xii) The assay and method/process developed in the present invention provides the clinicians and diagnostic laboratories with a simple, rapid and affordable method/process for PoC based in vitro diagnosis of disease and early monitoring of MCI, dementia, or AD.

Therefore, in the present invention, the assay method/process is re-designed to provide more simpler, rapid and costeffective approach for PoC in vitro assay method/process that eliminates the requirement of a PoC-chip component. The re-designed PoC assay in the present invention can be performed directly in the reaction tube and the detection signals can be directly monitored in real-time in free solution. This is accomplished by replacing the PoC-glass chip platform in previous invention with free, colloidal, and non-magnetic QDs-Abl-(x) in the present invention for direct signal detection in the reaction solution in real-time without any additional isolation/purification steps.

In this invention, it is aimed to develop a rapid and simple immuno-optomagnetic point-of-care (PoC) in vitro assay/process using multiple ("four") model disease biomarkers, such as Aβ1-40, Aβ1-42, tau-protein, and BDNF that are the indicators of Alzheimer's disease. These biomarker proteins are pathogenic amyloid fibrils that accumulate inside the human body during the course of aging, damage the brain and give rise to cerebral amyloid angiopathy, neuronal dysfunction and cellular toxicity that constitute the recognizable features of dementia, mild cognitive impairment and Alzheimer's disease. The in vitro immuno-optomagnetic assay/method developed utilizes a combination of multimodal features of bioactivated non-magnetic, colloidal/free QD nanocrystals as well as magnetic quantum dots (MQDs) to generate analyte-specific real-time quantitative/semi-quantitatively measurable signals for visible detection and quantification of multiple biomarker levels in serum.

The multiplexed biomarker detection is carried out using a combination of contrasting colored non-magnetic, fee/colloidal QDs and MQDs in a small microtube with a few tens of microliters volume. The assay process/method involves the following steps;
(i) Capturing of freely suspended serum analytes 'z' = [(1) Aβ1-40, (2) Aβ1-42, (3) tau-protein, and (4) BDNF] by bioactivated non-magnetic QDs-Ab1-(x) to form a primary Complex-1, where 'x' is bioreceptor antibody specific to 'z'.
(ii) The Complex-1 is then sandwiched on bioactivated MQDs-Ab2-(x) that captures the 'z' from serum sample to form a secondary Complex-2 (QDs-Ab1-(x)-z-(x)-Ab2-MQDs).
(iii) The multi-modal functionality of MQDs enabled rapid magnetic segregation of Complex-2 from non-magnetic QDs-Ab1(x) in serum sample.
(iv) The detection of diagnostic signal originates from the color change after binding of an analyte with two different colors (Wavelengths) of biofunctionalized MQDs (λ1) and bioactivated QDs (λ2) to give rise to a new color (λ1:λ2) of mixture.
(v) The resulting visible color and intensity change with Complex-2 occurred in real-time within 10-20 min that enabled rapid semi-quantitative and quantitative estimation of the biomarker analytes present in the free-serum solution, whose concentration-dependent change is visible to a naked eye in real-time.
(vi) The dynamic range of detection using the developed PoC assay for analytes (1) Aβ1-40 = 0~5 ng mL⁻¹, (2) Aβ1-42 = 0.31~10 ng mL⁻¹, (3) tau-protein = 0.07~1.25 ng mL⁻¹ and (4) BDNF protein = 0.31 to above 10 ng mL⁻¹. This range window of the levels of biomarkers enables determining the risk of the Alzheimer's disease.

The primary (Abl) or a second (Ab2) antibody in this invention is not limited to a single protein biomarker or disease but also possibly extended to a variety of biomolecules, receptors, pathogenic bacteria, virus, DNA/RNA, environmental contaminants, pesticides or drugs which are generally described as analyte specific reagents in the present invention.

The main difference in the present invention compared to our previous invention of (PCT/TR2020/050115) are the following:
(i) The newly designed PoC in vitro assay method/process does not require PoC-glass chip platform for analyte detection.
(ii) Instead of PoC-chip, the present invention requires bioactivated free/colloidal and non-magnetic QDs-Abl-(x) along with MQDs-Ab2-(x) to directly generate fluorescence in the reaction microtube. This approach enabled reducing the cost and time for assay performance.
(iii) Detection of signal is directly visualized or measured from the reaction tube in real-time as opposed to our previous invention, in which the Complex-2 had to be first isolated/purified before applying on PoC chip platform to be able to later detect the signal that demands more time and additional step.
(iv) The PoC in vitro assay in present invention is less time consuming and designed to deliver signal directly in a microtube in real-time within 10~20 min as compared to our previous invention, where the signal generation took place at least at 30 min of sample application.

The developed in vitro immuno-optomagnetic PoC assay method/process potentially create a new opportunity and approach to clinical diagnosis of chronic diseases and generate a new market in healthcare sector. The proposed invention provides an alternate to current practices for the diagnosis of Alzheimer's disease that are not only expensive but also time consuming and difficult for elderly patients to frequent hospital visits. The PoC assay method/process designed in this invention, therefore potentially revolutionizes the elderly care by enabling easy and early disease diagnosis and monitoring options to track risk markers in serum or body fluids of suspected elderly individuals.

The re-designed PoC assay in the present invention can be performed directly in the reaction tube instead of using a chip and the diagnostic signals can be directly monitored in real-time. This is accomplished by replacing the PoC-glass chip platform in previous invention with non-magnetic QDs-Abl-(x) in the present invention for direct signal detection in the reaction solution without having to follow any additional step.

The main components in the present invention are as follows;
(a) The whole PoC in vitro assay method is carried out in a small microtube containing free solution with a few tens of microliters volume.
(b) This PoC in vitro assay required; (i) bioactivated non-magnetic QDs of one color that form **Complex-1** with analyte and; (ii) multifunctional bioactivated MQDs of a different color that sandwich with **Complex-1** to form **Complex-2.** However, the MQDs here are similar to those used in our previous invention except that with a different bio-receptor, which also form crucial components in the present invention.
(c) The main difference however in the present invention is that the newly designed PoC in vitro assay does not require PoC-glass chip platform for analyte detection. Instead, bioactivated non-magnetic QDs directly generate fluorescence signal by sandwiching on "Complex-2" in real-time in free solution.
(d) Detection of signal can be directly visualized or measured from the reaction tube in real-time as opposed to our previous invention, where the Complex-2 had to be first incubated on PoC chip platform.
(e) The present PoC in vitro assay is less time consuming and deliver results within 10-20 min as compared to that in our previous invention, where the signal generation took place after 30~60 min of sample application.

The advantages, including novelty / innovative aspects of proposed invention as well as superiority to prior art is summarized as follows:
(i) The proposed invention describes an equipment-free, rapid, sensitive and easy-to-use in vitro immunooptomagnetic assay that is cost-effective PoC diagnostic for detecting disease risk biomarker analytes in biological fluids (eg., serum). The method/process is also applicable but not limited to other biofluids (blood, urine, saliva, sweat, CSF or interstitial fluid), water and environmental samples.
(ii) The present invention combines the multi-modality features of both non-magnetic and magnetic QDs to generate a sensitive immuno-optomagnetic signal that is visible to naked eye for rapid and semi-quantitative as well as quantitative analysis that saves time and cost. The signal generated is visible to a naked eye in real-time after exposing the tubes to a UV-torch.
(iii) The immuno-optomagnetic nanomaterials made of bioactivated non-magnetic and magnetic quantum dot nanocrystals that provided unique nanoarchitectures and multiplexing with their optical and magnetic properties that also carry biological specificity allowing real-time signal generation, visualization with specificity to target analytes. The detection of diagnostic signal originates from the color change after binding of an analyte with two different colors (Wavelengths) of biofunctionalized MQDs (λ1) and bioactivated QDs (λ2) give rise to a new color (λ1:λ2) of mixture.
(iv) The in vitro assay designed is carried out in a small volume of a few tens of microliters in a microtube, which is most desired in clinical diagnosis for screening multiple analytes from the same sample (multiplexing). (v) Diagnostic signal from the immuno-optomagnetic reaction tubes can be directly visualized by flashing with a UV-LED torch for a rapid semi-quantitative analysis.
(vi) The present invention related to in vitro immuno-optomagnetic PoC assay method/process does not required laboratory equipment or trained personnel to perform the assay, unlike most existing tools and techniques used for the same purpose, including tissue biopsy tests, ELISA tests, MRI, PET/CAT scans that makes rapid disease diagnosis extremely difficult, expensive, and time-consuming. Therefore, the novel method described in present invention has advantages of speed, sensitivity, selectivity, and cost-effectiveness that is lacking in current approaches, existing tools and techniques.

This invention will therefore provide a versatile tool for clinicians, physicians, or surgeons to rapidly and accurately identifying elderly population who may be at high risk. It is also possible that PoC based detection assay will facilitate monitoring the progression of MCI, AD or dementia. Multiplexed detection by the PoC tests potentially also ensures the quality, accuracy and performance of in vitro diagnostics, which will pave the way for novel health monitoring at home and add value to personalized medicine.

### Definition of the Figures Describing the Invention

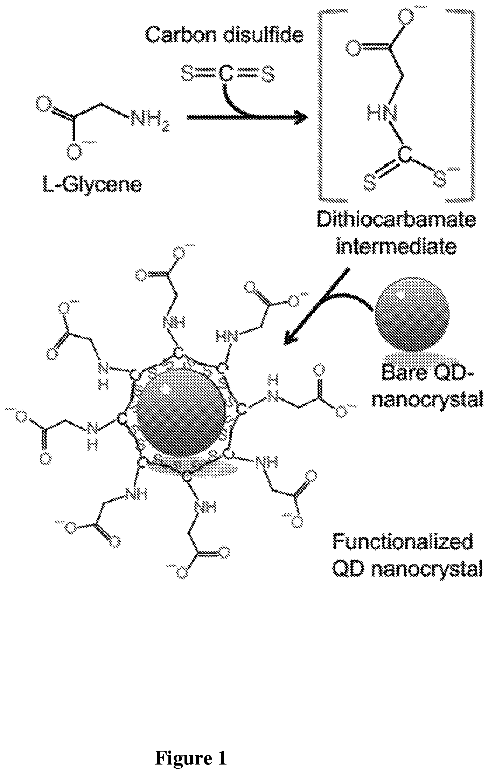

**Figure 1****:** Ligand exchange reaction between colloidal organic soluble quantum dots and poly-glycine capping to obtain highly water-soluble quantum dots.

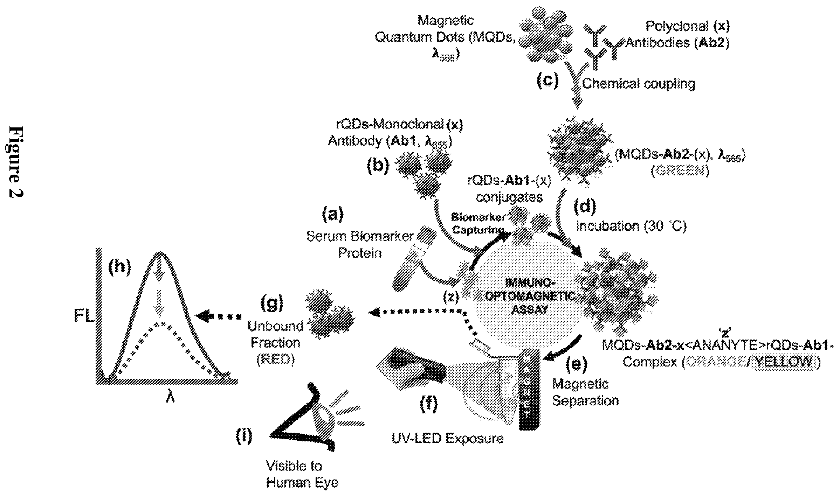

**Figure 2****:** Schematic illustration of in vitro immuno-optomagnetic assay designed for in-vitro screening for binding and specificity using model MQDs-Ab2-b for Ab1-42 protein detection. The term 'x' in scheme is assigned to either of the following: (a) anti-Ab1-40 antibody, (b) anti-Ab1-42 antibody, (c) anti-tau protein antibody, and (d) anti-BDNF antibody. The sign 'z' is assigned to either of the following: (1) Aβ1-40 protein analyte, (2) Aβ1-42 protein analyte, (3) tau-protein analyte, and (4) BDNF protein analyte. The scheme shows sequential steps involved in the inventive process as follows: (a) Serum sample containing antigen is mixed with (b) Non-magnetic QDs-Ab1-(b) (shown red) specific to Ab1-42 antigen, which then forms complex with the following, (c and d) green MQDs-Ab2-b carrying a second antibody that bind to same antigen from a different epitope. (e) Capturing of non-magnetic QDs-Ab1-(b) complex that gave rise to change in color from green to yellow (red + green = yellow), (f) Magnetic separation of MQDs-Ab2-b+antigen+Ab1-QDs-Ab1-b is visualized after UV-excitation and visible signal detection from reaction tubes. (g) Unbound or free antigen/non-magnetic QDs-Ab1-(b) were separated for measuring residual fluorescence using a spectrofluorometer or directly visualized after the UV exposure. (h) The fluorescence spectra are taken to calculate the quantitative levels of excess/unbound QDs-Abl-(x) fraction using fluorescence intensity changes. The fluorescence intensity of bound Complex-1 was calculated from the measured residual fluorescence intensity. (i) Fluorescence color change and signal is directly visualized by a naked human eye in real-time.

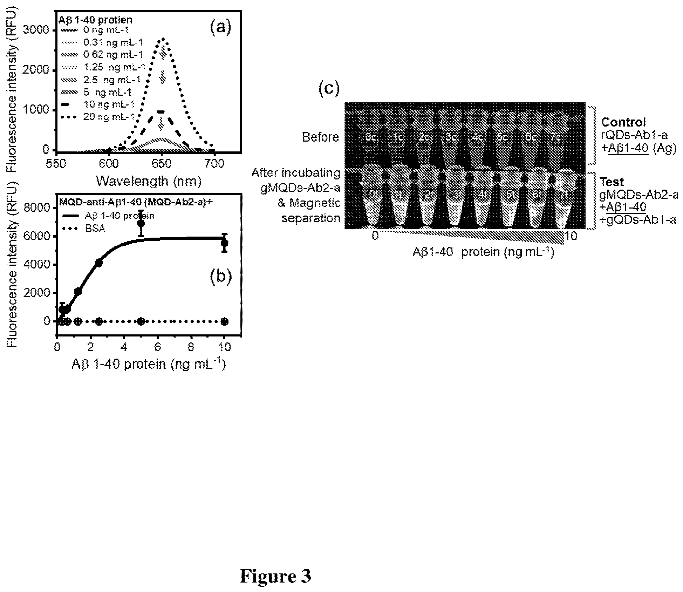

**Figure 3****:** (a) Changes in fluorescence intensity observed from fluorescence spectra of test samples calculated for bound fractions. (b) Non-linear regression analysis with fluorescence data at 655 nm showing binding saturation occurred at 5 ng mL-1 of analyte Aβ1-40 protein, while there was no binding occurred with non-specific BSA protein indicating specificity of gMQDs-Ab2-a and rQDs-Ab1-a. (c) Images of control and test reaction tubes captured before (top row) and after binding followed by magnetic separation (bottom row).

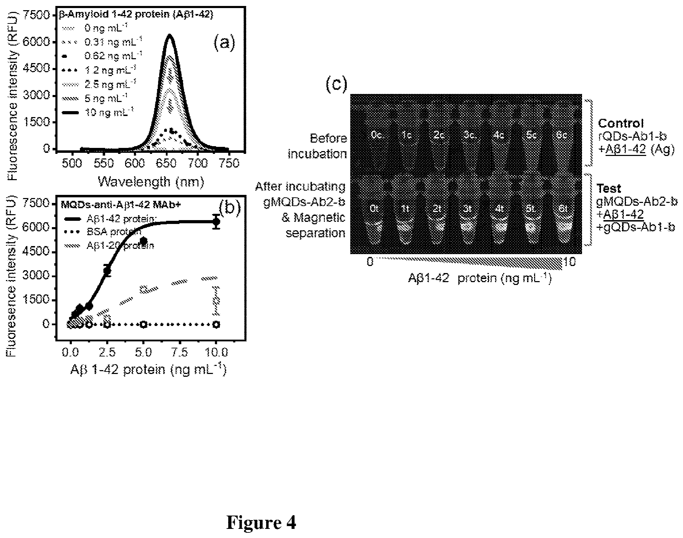

**Figure 4****:** (a). Changes in fluorescence intensity observed from fluorescence spectra of test samples calculated for bound fractions. (b) Non-linear regression fit of fluorescence intensity data extracted at a wavelength (655 nm) that showed no binding with BSA but partially bind to an analogous protein Aβ1-20 indicating the specificity of the reaction to Aβ protein fragments. (c) Images of reaction tubes captured after incubation of control and test reaction tubes and after magnetic separation showing clear capturing of Aβ1-42 analyte that saturated at 10 ng mL-1.

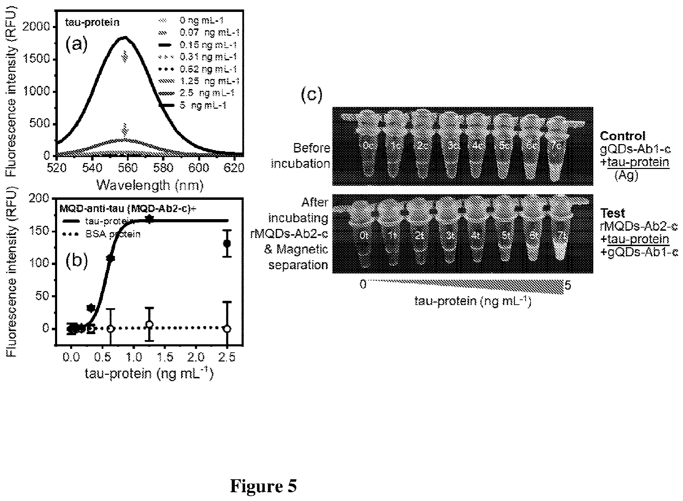

**Figure 5****:** (a) Changes in fluorescence intensities from fluorescence spectra of residual samples from reaction tubes after magnetic separation. (b) Calculated bound fluorescence intensities with varying analyte concentrations showing saturation at 1.25 ng mL-1 tau-protein with no non-specific binding with BSA. (c) Images of reaction tubes captured showing controls containing green non-magnetic QDs-Ab1-c with respect to increasing concentrations of analyte (tau-protein) from 0 (blank) to 5 ng mL-1 tau-protein and after incubation with constant amounts of red-MQDs-Ab2-c followed by magnetic separation showing capturing of analyte and saturation.

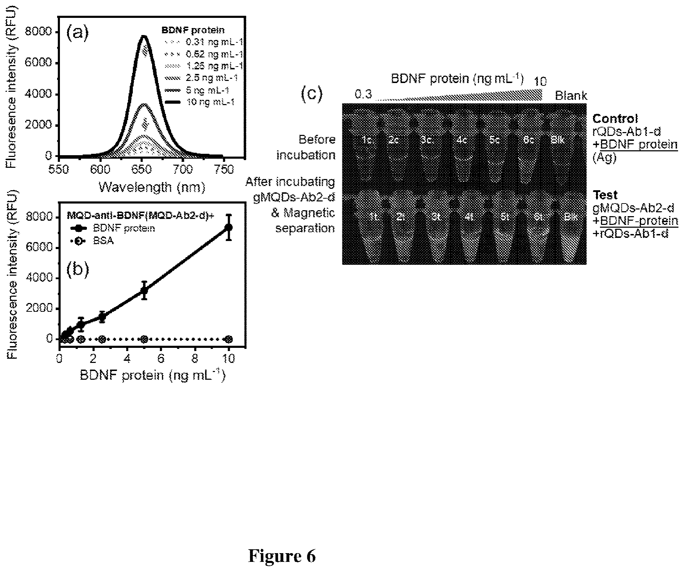

**Figure 6****:** (a) Fluorescence spectra showing changes in intensities linearly with respect to BDNF concentrations, and (b) Linearly increasing fluorescence intensities corresponding to increasing BDNF concentrations with no non-specificity with BSA. (c) Images of reaction tubes showing control samples with analyte BDNF pre-captured by red non-magnetic QDs-Ab1-d forming Complex -1, and the tests samples showing complete binding of Complex-1 on MQDs-Ab2-d forming Complex-2 that was magnetically separated within the free solution.

### Detailed Description of Invention

The present invention describes an in vitro point-of-care (PoC) immuno-optomagnetic assay method/process for rapid and sensitive detection of multiple protein biomarker/s for Alzheimer's disease in free solution. The developed method utilizes a combination of non-magnetic quantum dots (QDs) and magnetic quantum dots (MQDs) that captures serum analytes to display change in color that is directly visible to a naked eye within 10~20 min.

A method for detecting and quantifying of a target analyte in a test sample for central nervous system disease comprises following steps of:
- Providing free/colloidal bioactivated multi-colored CdSe/CdS/ZnS non-magnetic quantum dot (QD) functionalised with a primary analyte specific reagent,
- Providing a plurality of optomagnetic magnetic quantum dots (MQD) functionalised with a second analyte specific reagent, capable of binding to analyte followed by the primary analyte specific reagent,
- Providing both primary analyte specific reagent and second analyte specific reagent capable of binding to a common target analyte from different sides,
- Contacting said non-magnetic quantum dot (QD) with a target analyte and forming QD nanocrystals-primary analyte specific reagent-target analyte complex which is Complex 1,
- Forming a sandwich Complex 2 between said Complex 1 and magnetic quantum dots (MQD) functionalised with a second analyte specific reagent,
- Detection of diagnostic signal originates from the color change after binding of an target analyte with two different colored (Wavelengths) of biofunctionalized MQDs (λ1) and bioactivated QDs (λ2).
- Visiualizing of diagnostic signal generated by a naked eye in real-time using a UV-torch.

The present invention goes a step forward and extends the capabilities of previous immuno-optomagnetic PoC assay by following advancements:
In the current invention, the multi-colored non-magnetic CdSe/CdS/ZnS quantum dots (QDs) and magnetic quantum dots (MQDs) were "re-purposed" to develop a new equipment-free "solution phase PoC in vitro assay".
(i) The main difference between the current invention and our previous invention of PCT/TR2020/050115 is that the current invention does not require PoC-chips made of glass substrate for detecting the signal. Instead of PoC-chip, the present invention utilized free/colloidal and non-magnetic QDs of different colors to generate signal directly in the solution phase. The present invention describes multiple biomarker detection (multiplexing) using different colored QDs and MQDs
(ii) The whole PoC in vitro assay method is carried out in a small microtube containing free solution with a few tens of microliters volume as illustrated in Scheme 2(a-g).
(iii) This PoC in vitro assay required a combination of; (i) bioactivated free/colloidal and non-magnetic QDs-Ab-(x) of one colors and (ii) multifunctional bioactivated MQDs-Ab2-(x) of a different colors that bind to same analyte protein.
(iv) These MQDs in the present invention are similar to those MQDs used in our previous invention, but with different receptor antibodies that also constituted a crucial component in present invention.
(v) The extended invention method/process is demonstrated for the multiplexed detection of a panel of 'four' different Alzheimer's disease (AD) biomarkers (z) as model protein analytes for a single disease type (AD). The four biomarker proteins 'z' tested in the present invention are the variants of amyloid β (Aβ) proteins, such as (1) Aβ1-40 and (2) Aβ1-42, along with two distinct biomarkers, such as (3) tau-protein and (4) Brain-Derived Neurotrophic Factor (BDNF) in human serum.
(vi) Instead of PoC-chip, the present invention requires bioactivated free/colloidal and non-magnetic QDs-Abl-(x) to directly generate fluorescence signal directly in reaction microtube.
(vii) Here, free/colloidal, and non-magnetic QDs-Abl-(x) first capture analyte protein 'z' from the serum to form a "Complex-1" of QDs-Ab1-(x)-z
(viii) This Complex-1 is then sandwiched with MQDs-Ab2-(x) by immuno-reaction to form a "Complex-2" of MQDs-Ab2-(x)-z-(x)-QDs-Ab1 in a microtube free solution. The Ab1 and Ab2 antibodies are designed to bind the same antigen from different epitopes.
(ix) Detection of signal is directly visualized or measured from the reaction tube in real-time as opposed to our previous invention, where the Complex-2 had to be first isolated/purified before applying on PoC chip platform to be able to detect the signal.
(x) The PoC in vitro assay in present invention is less time consuming and designed to deliver signal directly in a microtube in real-time within 10~20 min as compared to our previous invention, where the signal generation took place at least at 30 min of sample application. The visible signal is generated directly in the reaction microtube within 10~20 min after the sample addition with dynamic detection ranges of 0.31~5 ng mL-1 (Aβ1-40), 0.31~10 ng mL-1 (Aβ1-42), 0.07~1.25 ng mL-1 (tau protein) and from minimum 0.31 to above 10 ng mL-1 (BDNF protein).
(xi) The entire PoC in vitro assay method/process of our present invention is schematically illustrated in Scheme 2(a-g).

In this invention, we developed a rapid and simple point-of-care (PoC) in vitro assay/process using multiple ("four") disease biomarkers ('z') as models, where 'z' is either of the following biomarker proteins; (1) Aβ1-40, (2) Aβ1-42, (3) tau-protein, and (4) BDNF that are the indicators of Alzheimer's disease. The developed point-of-care in vitro method/process potentially replaces current practices for diagnosis of Alzheimer's disease that are time-consuming tests (see Annex. 1), or rely on heavily centralized facilities, such as MRI at hospitals at the stage when the individual already had visible signs of Alzheimer's disease, dementia, or mild cognitive impairment (MCI). The PoC assay method/process designed in this invention has the potential to revolutionize elderly disease diagnosis by utilizing serum or body fluids to rapidly detect for disease risk biomarkers.

### 1. Synthesis of single-color core-shell-shell CdSe/CdS/ZnS nanocrystals

### 1.1. Synthesis of green CdSe/CdS/ZnS nanocrystals

Single color green CdSe core nanocrystals with gradient CdS and ZnS shell-shell were synthesized using a previously described method [16]. First, Se-S solution was prepared in a glovebox by dissolving 0.316 g (4 mmol) selenium powder and 0.128 g (4 mmol) sulfur powder in 3 mL TOP and placed in a desiccator under vacuum until use. In a separate three-necked flask, 0.025 g (0.2 mmol) CdO, 0.87 g (4 mmol) zinc acetate, 2.75 mL oleic acid and 10 mL octadecene were added and the flask was heated to 150 °C for 1 h under vacuum for degassing. This was followed by introducing argon gas into the flask from an inlet connected to a condenser, while vacuum tube connected at the outlet and the reaction temperature was raised to 310 °C. After stabilizing the temperature to 310 °C, 1.5 mL of above Se-S solution was quickly injected to the solution while maintaining the temperature to 304 °C and waited for 20 min for reaction to complete. This procedure was optimized to obtain a single color (green) nanocrystals that were subjected to purification.

### 1.2. Synthesis of red/orange CdSe/CdS/ZnS nanocrystals

Single colored red CdSe core with gradient CdS and ZnS shells of nanocrystals were synthesized as described previously [17]. Here, instead of mixing the Se and S solutions together, they were prepared separately in a glovebox. For this, selenium solution was prepared by dissolving 0.158 g (2 mmol) Se powder in 0.89 mL (0.74 g) trioctylphosphine (TOP), while the sulfur solution was prepared separately by dissolving 0.192 g 23 mmol sulfur powder in 0.45 mL (0.37 g) TOP. These solutions were placed in a desiccator under vacuum until use.

In a separate three-necked flask, 0.09 g (0.7 mmol) CdO, 0.27 g (1.5 mmol) zinc acetate, 3.6 mL oleic acid and 15 mL octadecene was poured and degassed by heating at 150 °C for 1 h under vacuum. The argon flow at the condenser inlet was turned on and raised the reaction temperature in flask to 300 °C. Once the temperature was stabilized, 0.1 g (1.2 mmol) of Se solution was quickly injected to the reaction mixture using a syringe needle, after 90 seconds, 0.018 mL dodecanethiol was added gradually by slow injection and the reaction was allowed to continue for 15 min. Lastly, 0.29 g sulfur solution prepared previously was injected into the reaction flask and waited 15 min for reaction to complete and removed the flask for cooling the reaction mixture.

About 15 mL of the reaction mixture containing red CdSe/CdS/ZnS nanocrystals was diluted with equal volume of chloroform and precipitated by adding 45 mL acetone and centrifuged for 15 min at 4500 rpm. The supernatant was removed, and the pellet was re-dissolved in 2 mL chloroform and re-precipitated by adding 6 mL acetone, centrifuged for 5 min at 4500 rpm and the process was repeated at least 8 times. Finally, the precipitate was dried at 60 °C overnight and the dried powder yielded 73 mg of CdSe/CdS/ZnS nanocrystals that was subjected to ligand exchange as described above.

The entire reaction mixture containing green/red nanocrystals and impurities were divided into several 1 mL aliquots. Each aliquot was diluted by adding 10 mL chloroform and the nanocrystals were precipitated by mixing with 30 mL acetone non-solvent and centrifuged for 2 h at 4500 rpm. The supernatant was collected in a separate tube that contained residual nanocrystals for further precipitation, while the pellet was dissolved in 2 mL chloroform and precipitated again by adding 6 mL acetone and centrifuged for 2 h at the same speed. This process was repeated 10 times and the final precipitate obtained was dried at 60 °C. The dried nanocrystals were subjected to ligand exchange process for surface functionality and water solubility as described below.

### 1.3. Ligand exchange and QDs stabilization

Single color green or red QD nanocrystals synthesized above were subjected to surface coating through ligand exchange process using a combination of methods described previously [18, 19]. Briefly, synthesized pure and dried coreshell- shell green and red QD nanocrystals (2.85 mg each) were separately dissolved in 1 mL chloroform. In a separate vial, 0.25 g of L-glycine was dissolved in 5 mL deionized water and mixed with 0.2 mL of carbon disulfide and vortexed.

At this stage, a milky solution of functionalized QD nanocrystal was obtained (Scheme 1) which was added with 1 mL (2.85 mg in chloroform) of green or red core-shell-shell QD nanocrystals and the mixture was stirred for 24 h on a magnetic stirrer at room temperature. During this period, phase transfer of the QDs occurred from organic phase to aqueous phase following coating with a thin layer of dithiocarbamate and a hydrophilic poly-glycine layer, making the QDs more stabilized and colloidal in aqueous solution. The transfer of QDs to aqueous phase was visibly observed under the UV light as a conformation of ligand exchange reaction.

The nanocrystals from aqueous phase were aspirated and purified by diluting with deionized water in 1:4 ratio and precipitated by adding 20 volumes of acetone followed by centrifugation at 10000 rpm for 30 min for green QDs and 5000 rpm for 5 min for red QDs, respectively. The precipitate was then re-dispersed in aqueous buffer (PBS, pH 7.4). The water-soluble core-shell-shell QDs were later stored at 4 °C until use.

### 2. Synthesis of magnetic Fe₃O₄ nanoparticles (MNPs)

Magnetic Fe₃O₄ nanoparticles (MNPs) were synthesized using a previously reported co-precipitation method [20] and were size-fractionated at varying relative centrifugal force (RCF, g). Briefly, 0.25 mL (Fe+2) of 2 M FeCl2·4H2O in HCl was mixed with 1 mL of 1.1 M FeCl3·6H2O (Fe+3) in deionized water for 15 min and co-precipitated under alkaline conditions. The salt solution was heated for 10 min at 80 °C and the co-precipitation of Fe+2 and Fe+3 was initiated by slow dropwise addition of 3 mL of 3 M ammonia solution. MNPs obtained were separated with the help of a magnet and the MNPs residue was washed with double deionized subjected to gravity (g) fractionation at varying RCFs.

Gravity fractionation of MNPs was carried out and isolated different nano-sizes by applying RCF from 500~14500'g for 5~10 min, which enabled fractionating different nano-sized MNPs. Larger sized MNPs fraction that sedimented at 500~14500'g within 5 min were removed and only those fractions that were sedimented at 16000' g in 5 min were collected whose size ranged between 5~10 nm. These bare MNPs were subjected to stabilization and images were taken using an ultra-high-resolution TEM (JEOL JEM-ARM200CFEG UHR-TEM equipped with STEM, Cs corrected STEM and EDS).

### 2.1. MNPs stabilization

As-synthesized bare MNPs were suspended in freshly prepared 4.5 mL of 0.3 M ammonia solution and the MNPs were dispersed by sonication using a probe ultrosonicator for 1 h. The colloidal MNPs were washed with five exchanges of 1 mL of deionized water followed by 3 min magnetic separation. The washed bare MNPs were dispersed in 4.5 mL of 50 mM L-aspartic acid and mixed by stirring vigorously for 6 h until the solution pH=2-3. The reaction pH was raised to 10~11 by dropwise addion of 3 M ammonia solution and the excess non-adsorbed aspartic acid was removed in successive magnetic separation and washing five-times with 2 mL dilute ammonia solution (0.3 M). Finally, the aspartic acid-stabilized Fe₃O₄ magnetic nanoparticles (Asp-MNPs) were stored in same solution until further use for chemically coupling with QD nanocrystals.

### 3. Synthesis of multi-colored magnetic quantum dots (MQDs)

Polymer stabilized green/red CdSe/CdS/ZnS core-shell-shell QD nanocrystals carrying free carboxyl functionality were used for synthesis of green/red MQDs. Green MQDs (gMQDs) or red MQDs (rMQDs) were synthesized using non-magnetic water-soluble green QDs (gQDs) and red QDs (rQDs) as follows. First, 200 µL (0.57 mg) of water-soluble green QDs were suspended in 500 µL deionized water and vortexed for 5 min. To this, 100 µL of freshly prepared 150 mM of 1-ethyl-3-(3-dimethylaminopropyl)carbodiimide hydrochloride (EDC) and incubated for 5 min followed by addition of 100 µL of 300 mM N-hydroxysuccinimide (NHS) and the reaction mixture was again incubated for 5 min under static condition. The resulting EDC-activated bright fluorescent nanocrystals were quickly mixed with 1 mL of 10 mg/mL of Asp-MNPs (5~10 nm) suspension in deionized water and incubated for 2 h for covalent coupling under vigorous shaking at room temperature. The resulting reaction mixture containing hybrid MNPs@CdSe/CdS/ZnS nanoparticles (magnetic quantum dots, MQDs) were magnetically separated and washed by resuspending in PBS solution, pH 7.4. Coating of MNPs on nanocrystal surfaces was confirmed visibly after magnetically separating gMQDs and rMQDs separately that appeared brightly illuminating magnetic nanoparticles upon exposure to UV irradiation at 365 nm, which accompanied by disappearance of fluorescence from the supernatant solution.

### 4. Bio-functionalization of QDs with polyclonal (Ab1) and MQDs with monoclonal antibodies (Ab2)

Red/green (r/g) QDs and MQDs conjugated with a specific antibody (Ab1 and Ab2) is termed as QDs-Ab1 and MQDs-Ab2, and the type of specific antibodies 'x' is assigned to any of the following antibody types:
(a) anti-Aβ1-40 antibody
(b) anti-Aβ1-42 antibody and
(c) anti-tau protein antibody
(d) anti-BDNF antibody

The above four distinct antibodies are plurally termed as 'x' that represent either of the above three types of Abs for convenience. For example, MQDs-Ab2(x) represents MQDs conjugated with either of the above antibody types (a, b, c, or d), for instance rMQDs bioconjugated with anti-Aβ1-40 antibody is termed as rMQDs-Ab-a.

Bioconjugation was carried out using 20 mg of different colored (r/g) MQDs such as green (λ565, gMQDs) or red (λ655, rMQDs) MQDs. Similarly, bioconjugation of 4.32 mg different colored non-magnetic QDs was carried out, such as green-QDs (λ565, gQDs) or red-QDs (λ655, rQDs). Here, bioconjugation of antibodies specific to biomarker protein was designed to pair with distinct colored magnetic and non-magnetic QDs (MQDs:QDs). For instance, green MQDs (gMQDs) always paired with non-magnetic red QDs (rQDs) and vice versa.

Bioconjugation was carried out for non-magnetic QDs, for instance 4.32 mg of rQDs (λ655,) or oQDs(λ585) or 20 mg of gMQDs (λ565) with free carboxyl-functionality were dispersed each in 100 µL of deionized water. To this, 100 µL mixture containing 50 mM EDC and 100 mM NHS in deionized water was mixed and incubated for 5 min. The surface activated gMQDs were magnetically separated, while surface activated non-magnetic rQDs were centrifuged at 14000 rpm for 5 min and the supernatant was discarded. The resulting nanocrystals were resuspended in 400 µL PBS solution (pH 7.4) and washed thrice by magnetic separation and centrifugation for gMQDs and rQDs, respectively. The activated gMQDs and rQDs (or rMQDs and gQDs) pellets were mixed each with 10 µL of 1 mg mL-1 polyclonal and monoclonal antibodies, respectively and incubated at 4 °C for 5 h. After incubation, the g/rMQDs-Ab2-(x) bioconjugates (MQD-Ab2-a, MQD-Ab2- b, MQD-Ab2-c, and MQD-Ab2-d) were separated by magnetic separation and non-magnetic g/rQDs-Ab1(x) (QD-Ab1-a, QD-Ab1-b, QD-Ab1-c, and QD-Ab1-d) were separated by centrifugation at 14000 rpm for 5 min and respective supernatants were discarded. The above two processes were repeated thrice to wash bioconjugates (QDs-Ab1(x) and MQDs-Ab2-(x)) that were subjected to blocking step.

In addition, bare Asp-MNPs were conjugated with polyclonal antibodies (MNPs-x) as described above for MQDs-Ab2-(x). The free-functional groups on bioconjugates were blocked by adding 400 µL of 5% bovine serum albumin (BSA) in PBS solution (pH 7.4) in each reaction tube and incubated at 4 °C for 2 h. Finally, the bioconjugates were separated and washed with PBS in three cycles following magnetic separation or centrifugation, and finally resuspended in 400 µL of same buffer and stored at 4 °C until use.

### 4.1. Blocking nonspecific sites on QDs-Ab1(x) and MQDs-Ab2-(x) surfaces

The purified bioconjugates with any free active functional groups were blocked by incubating with 5% BSA in PBS solution (pH 7.4) containing 0.05% tween 20 (blocking buffer) for 30 min. For negative controls, non-magnetic QDs and MQDs were also incubated with only BSA to prepare QD-BSA or MQD-BSA protein conjugates under standard conditions. Finally, all bioconjugates were washed with PBS in three cycles following magnetic separation or centrifugation for magnetic and non-magnetic QD nanoparticles, respectively and resuspended in 400 µL of same buffer. All bioconjugates were again divided into several 10 µL aliquots and stored at 4 °C until use for in-vitro immuno-optomagnetic detection assay.

### 5. Preparation of analyte protein spiked serum samples

Normal serum from human plasma (2.0 mL, male AB plasma, Sigma-Aldrich) was first treated by incubating with 10 mg of non-fluorescent MNPs-Ab1-(x) for 1 h to remove pre-existing or background target protein that may be present in the serum. The resulting MNPs-Ab1 were magnetically separated from target protein analyte-free serum. The above process was repeated at least thrice with same serum but by incubating with a 10 mg of fresh aliquot of MNPs-Ab1-(x).

Finally, the serum sample devoid of any analyte protein traces was used for the preparation of known levels of analyte protein (y) spiked serum samples. Here, the analyte protein (y) generally refers to any of the following analyte proteins; (i) Aβ1-40 (Invitrogen), (ii) Aβ1-42 (Abcam, Cambridge, MA), (iii) tau-protein (R&D Systems), and (iv) Brain Derived Neuritic Factoris (BDNF, Fitzgerald). The pure analyte proteins (y) were first reconstituted as per the manufacturer's instructions and diluted with analyte-free serum to obtain a working stock of 20 ng mL⁻¹ of specific target protein, and this sample was again serially diluted using analyte-free serum to obtain a series of concentration of analyte protein (0~20 ng mL⁻¹). For negative controls, serum samples were spiked with BSA protein instead of target analyte protein under identical conditions as described above. These analyte protein/BSA spiked serum samples were utilized for in-vitro immuno-optomagnetic sensing assays.

### 6. Point-of-care in vitro immuno-optomagnetic test for detecting serum biomarker protein

In vitro screening for specific binding and specificity of MQDs-Ab2-(x) was carried out using antigen-free serum sample "spiked" with known concentrations of respective antigens (Aβ1-40, Aβ1-42, tau protein or BDNF). Here a series of varying antigen concentrations (0~20 ng/mL) for each type of antigen was incubated with constant amounts of nonmagnetic QDs-Ab1-(x) previously coated with '(x)' antibodies. The antigen-antibody complex on non-magnetic QDs were then presented to specific antibody coated MQDs-Ab2-(x) as shown in Scheme 2(a-e) as an example for Aβ1-42 detection. The non-magnetic QDs and MQDs were of different color for probing color changes post-incubation of antigens. The unbound or free non-magnetic QDs or free antigen can be easily detected by measuring the residual fluorescence of non-magnetic QDs to calculate the bound antigen in the sample upon magnetic separation of MQDs-Ab2-(x).

### 7. PoC Immuno-optomagnetic assay

Immuno-optomagnetic assay was carried out using a constant 15 µL (162 ng) of non-magnetic QDs-Abl-(x) for each reaction tube in a series of spiked serum samples containing 0, 0.31, 0.62, 1.2, 2.5, 5 and 10 ng mL-1 of antigen protein in replicates (*n* = 3) and incubated for 15 min at 30 °C. This reaction was then followed by mixing constant 15 µL (2 mg) of MQDs-Ab2-(x) conjugates to each reaction tube and the volume was adjusted to a final 80 µL using antigen-free serum and mixed. Here the term 'x' in non-magentic QD-Ab1-(x) and MQDs-Ab2-(x) is plurally identifies any of the following specific antibodies; Aβ1-40, Aβ1-42, tau protein or BDNF proteins. For negative controls, (i) BSA protein was used instead of antigen protein under identical conditions or (ii) swapped with non-antigen proteins. The reaction tubes were then incubated again at 30 °C for 15 min and the detection signal was directly observed by exposing the rection tube/s to the UV-LED (λ₃₆₅) torch, and the changes in fluorescence colors due to the formation of bioconjugates-antigen protein complex were recorded before and after incubation. Here, the non-magentic QD-Ab1-(x) first captures target antigen molecules to givie rise to a QD-Abl-(x)+target primary complex (Complex-1) in serum which is later presented to sandwich by immuno-reaction with green MQDs-Ab2-(x) conjugates. The resulting secondary complex (Complex-2) yields a tertiary color for instance, faint orange colored fluorescence to the reaction mixture from its original red and green. The resulting Complex-2 of MQDs-Ab2-(x)+target+non-magnetic-QD-Ab1-(x) formed is magnetically separated and a distinctive tertiary colored fluorescence from parent colors is measured or directly visualized using a naked eye in real-time (Scheme 2(a-g)). The residual fluorescence from the supernatant containing free non-magnetic QD-Ab1 is measured using a spectrofluorometer (Nanodrop 3300). This residual fluorescence provided an accurate measurement of the concentration of target biomarker/s (Aβ1-40, Aβ1-42, tau protein or BDNF proteins) captured by MQDs-Ab2-(a~d) in the reaction mixture. The fluorescence intensity of bound Complex-1 was calculated using the measured residual fluorescence intensity.

### 8. Specificity

Specificity of the immuno-optomagnetic assay was tested under identical conditions as described above, except that in place of the target protein, non-specific proteins, such as BSA or antigen swapping (e.g., non-specific and truncated antigen namely Aβ-20) was utilized.

### 9. Choice of target analytes for developing PoC in vitro assay/process and terminologies

In this invention, we aimed at developing in vitro PoC assay method/process using model multiple ("four") disease biomarkers, such as Aβ1-40, Aβ1-42, tau-protein, and BDNF that particularly represent for an Alzheimer's disease. The PoC assay/process designed is not limited to above four biomarkers but can also be plurally applicable to sensing various other disease biomarkers, pathogens, drugs, environmental contaminants, or DNA/RNA. The model biomarker proteins targets used in this invention, such as Aβ1-40, Aβ1-42 and related various amino acid length fragments are variants of Aβ proteins that are regarded as pathogenic amyloid fibrils. These fibrils are found to accumulate inside the human body during the course of aging, damage brain and cause cerebral amyloid angiopathy, neuronal dysfunction as well as cellular toxicity that are characteristics features of an Alzheimer's disease [21, 22]. Tau protein and BDNF also have crucial roles in the pathogenesis of Alzheimer's disease [23, 24].

For better clarity following terminologies have been adapted in this invention:
Red/green (r/g) freely suspended, colloidal and non-magnetic QDs were bioconjugated with specific antibodies are designated as QDs-Ab1. The magnetic quantum dots (MQDs) bioconjugated with a specific antibody is designated as MQDs-Ab2. The type of antibodies plurally/generally designated (termed) as 'x' that represent any one of the following antibody types depending upon specific assay carried out related to it:
(a) anti-Aβ1-40 antibody
(b) anti-Aβ1-42 antibody and
(c) anti-tau protein antibody
(d) anti-BDNF antibody

For example, MQDs-Ab2(x) represents MQDs conjugated with either of the above antibody types (a, b, c, or d), for instance rMQDs bioconjugated with anti-Aβ1-40 antibody is termed as rMQDs-Ab-a.

Similarly, serum biomarkers or analytes plurally designated as 'z', where z is either of the following four biomarker proteins that are representative indicators of Alzheimer's disease:
(1) Aβ1-40
(2) Aβ1-42
(3) tau-protein
(4) BDNF

Here, QDs-Abl-(x) and MQDs-Ab2-(x) bind to specific analyte 'z', for example.:
QDs-Abl-a and MQDs-Ab1-a always bind to same biomarker protein (1) Aβ1-40 but from different epitopes
QDs-Ab1-b and MQDs-Ab1-b always bind to biomarker protein (2) Aβ1-42 but from different epitopes
QDs-Ab1-c and MQDs-Ab1-c always bind to biomarker protein (3) tau protein but from different epitopes
QDs-Ab1-d and MQDs-Ab1-d always bind to biomarker protein (4) BDNF protein but from different epitopes

### 10. Point-of-care in vitro immuno-optomagnetic assay method/process for detecting serum biomarker protein

### 10.1. Design of PoC in vitro Immuno-optomagnetic assay

Immuno-optomagnetic assay was carried out using a constant 15 µL (162 ng) of non-magnetic QDs-Abl-(x) for each reaction tube in a series of spiked serum samples containing 0, 0.31, 0.62, 1.2, 2.5, 5 and 10 ng mL-1 of antigen protein 'z' in replicates (n = 3) and incubated for 15 min at 30 °C. This reaction was then followed by mixing constant 15 µL (2 mg) of MQDs-Ab2-(x) conjugates to each reaction tube and the volume was adjusted to a final 80 µL using antigen-free serum and mixed. Here the term 'x' in non-magentic QD-Ab1-(x) and MQDs-Ab2-(x) plurally identifies any of the following specific antibodies specific to analyte proteins 'z', where z = (1) Aβ1-40, (2) Aβ1-42, (3) tau protein or (4) BDNF proteins. For negative controls, (i) BSA protein was used instead of antigen protein under identical conditions or (ii) swapped with non-antigen proteins, for instance analogue Aβ1-20 protein. The reaction tubes were then incubated again at 30 °C for 15 min and the detection signal was directly observed by exposing the rection tube/s to the UV-LED (λ₃₆₅) torch, and the changes in fluorescence colors due to the formation of bioconjugates-antigen protein complex were recorded before and after incubation. Here, the non-magentic QD-Ab1-(x) first captures target antigen molecules to givie rise to a QD-Ab1-(x)+target primary complex (Complex-1) in serum which is later presented to sandwich by immuno-reaction with green MQDs-Ab2- (x) conjugates. The resulting secondary complex (Complex-2) yields a tertiary color for instance, faint orange colored fluorescence to the reaction mixture from its original red and green. The resulting Complex-2 of MQDs-Ab2-(x)+target+non-magnetic-QD-Ab1-(x) formed is magnetically separated and a distinctive tertiary colored fluorescence from parent colors is measured or directly visualized using a naked eye in real-time (Scheme 2(a-g)). The residual fluorescence from the supernatant containing free non-magnetic QD-Ab1-(x) is measured using a spectrofluorometer (Nanodrop 3300). This residual fluorescence provided an accurate measurement of the concentration of target biomarker/s ('z') captured by Complex-2 containing QDs-Abl-(x) and MQDs-Ab2-(x) in the reaction mixture (QDs-Ab1- (x)-z-(x)-Ab2-MQDs). The fluorescence intensity of bound Complex-1 was calculated using the measured residual fluorescence intensity, while the change in the fluorescence color is directly visualized by a naked eye in real-time.

### 11. Results

### 11.1. PoC in vitro binding and specificity test using rQDs-Ab1-a gMQDs-Ab2-a and serum analyte Aβ1-40 protein

To a series of spiked serum samples containing varying concentrations of known Aβ1-40 protein from 0 to 20 ng mL-1 was incubated each with a constant 162 ng (15 µL) of non-magnetic QDs-Abl-(x) and incubated for 15 min at 30 °C to form a Complex-1 as described in experimental methods. Each of these tubes were then added with a constant 2 mg (15 µL) of MQDs-Ab2-a to capture and sandwich with Complex-1 that gave rise to a Complex-2. This Complex-2 provided a distinctive fluorescent color which is different from the parent colors that was proportional to the analyte concentration (Aβ1-40 protein), which provided a semi-quantitative (visible to naked eye) and it was quantitatively estimated to determine the analyte Aβ1-40 protein concentration present in the test serum. Figure 3(a) shows fluorescence spectra of residual (unbound) QDs-Abl-a after magnetic isolation of Complex-2 from the reaction in real-time and Figure 3(b) shows dose-dependent response exhibiting saturation that was consistent to that observed from the residual fluorescence (Figure 3(a)). Figure 3(c) shows real images of reaction tubes (controls and tests) captured before and after binding reactions, respectively. Formation of Complex-2 containing hybrid structure made of MQDs-Ab2-a+Ab1-40-QDs-Ab1-a was magnetically isolated and the resulting transformation of primary color in to a tertiary yellow color is visible to a naked eye in real-time. Further, the visible changes in the intensity of the residual red-fluorescence coming from the free/excess QDs-Abl-a also enabled rapid semi-quantitatively visualize biomarker levels present in a given serum sample with reference to control. Specific changes with respect to analyte concentration was validated by observing the change in intensity of fluorescence from the fluorescence spectra and measured the residual fluorescence intensities as shown in Figure 3(b-c). The in vitro assay with MQDs-Ab2-a and QDs-Abl-a against varying concentrations of Aβ1-40 protein under specified standard conditions showed a detection range between 0.31~5 ng mL⁻¹ for Aβ1-40 with no non-specific binding when tested with a control BSA in place of Aβ1-40 under identical conditions (Figure 3(b), black line). The designed assay method/process was further extended to detecting other serum biomarkers of a particular disease (Alzheimer's disease) as a model. However, this assay method/process is not limited to those biomarkers tested in this invention but can be plurally applicable to sensing various other diseases, bacterial/viral pathogens, drugs, environmental contaminants, and DNA/RNA.

### 11.2. PoC in vitro binding and specificity test using gMQDs-Ab2-b, rQDs-Ab1-b and analyte Aβ1-42

In this invention, the designed assay/method for detecting Aβ1-40 was applied to another pathogenic variant of amyloid β (Aβ) protein biomarker, namely Aβ1-42 whose molecular structure differs by the presence of two additional amino-acids in Aβ1-42 compared to Aβ1-40. Both Aβ1-40 and Aβ1-42 are pathogenic amyloid fibrils that accumulate inside the human body during the course of aging, damage the brain and give rise to cerebral amyloid angiopathy, neuronal dysfunction and cellular toxicity that are associated with the Alzheimer's disease [21, 22]. Results of immuno-optomagnetic in vitro assay for sensing Aβ1-42 is shown in Figure 4(a-c). The dose-dependent relative changes in fluorescence intensity values with respect to controls was observed in the fluorescence spectral profile (Figure 4(a)). Figure 4(b) shows typical sigmoidal binding curve obtained after fitting the data in a non-linear regression equation with varying Aβ1-42 showed binding occurred at concentrations from 0.31 ng mL-1 until saturation at -10 ng mL-1 of Aβ1-42. We also tested MQDs-Ab2-b specificity with a non-specific BSA to ascertain that the signal is indeed originated from binding to analyte Aβ1-42 (Figure 4(b), black line). However, specificity test with a variant analogue protein could establish a strong relationship of developed assay specific to specific analyte. Therefore, an another amyloid β variant was introduced into the assay, namely Aβ1-20 which is a truncated 22-amino acid short of the original Aβ1-42 protein analyte that exhibited partial binding characteristics with gMQDs-Ab2-b and rQDs-Ab1-b. This result indicated that the designed assay is also applicable to detecting fragments of varying lengths of amyloid β protein fragments released from the brain in the serum. Figure 4(c) shows images of reaction tubes containing varying concentrations of Aβ1-42 protein binding to MQDs-Ab2-b at 0-min and 30-min intervals that can be visibly probed for change in color following magnetic separation. The binding of analyte is evidently visible to a naked eye from tubes within 15~20 min of the reaction.

### 11.3.In vitro binding and specificity assay using MQDs-Ab2-c and QDs-Ab1-c with structurally distinct proteins but represents for same disease (Alzheimer's disease)

Further extension of the developed immuno-optomagnetic assay method/process was applied to two distinct biomarker proteins, such as tau-protein and BDNF proteins that also represent as biomarkers of Alzheimer's disease.

Detecting a single biomarker for a particular disease presents potential risk of inaccuracy or false positive or false negative results. Therefore, detecting a panel of multiple biomarkers for a single disease for diagnosis maximizes the overall diagnostic accuracy in medical diagnosis. Therefore, in this invention, we extended the developed immunooptomagnetic in vitro assay method/process to other AD biomarkers, such as tau-protein and BDNF to enhance the assay performance in its accuracy for diagnosing the Alzheimer's disease.

### 11.4. In vitro binding and specificity assay using MQDs-Ab2-c and tau protein:

In this experiment, to distinguish from other experiments, the color of MQDs-Ab2-c and non-magnetic QDs were swapped. Instead of green MQDs, here we utilized red MQDs for distinctions from above two biomarkers. Figure 5(a) shows spectra of changes in residual fluorescence intensities obtained from samples after magnetic separation.

Sensitive and specific capturing of antigen is clearly visible from spectra until saturation at 1.25 ng mL⁻¹ tau protein which can be clearly observed form the relative fluorescence intensity values at λ₅₆₅ and the data were fitted with a non-linear regression equation that showed binding curve saturation in Figure 5(b). This signal was visibly seen directly in real-time from the reaction tubes within 15~20 min of sample incubation and magnetic separation (Figure 5(c).

### 11.5. In vitro binding and specificity assay using MQDs-Ab2-d, QDs-Ab1-c and BDNF protein:

Figure 6(b-c) show fluorescence spectra and data values plotted that showed linearity with increasing concentration up to 10 ng mL-1 BDNF. This result demonstrated that the binding efficiency can be further extended to a much broader concentration range because the linearity of curve fitted with a non-linear regression equation shows the system can be used for even higher concentrations. However, since the presence of BDNF at even a picogram level indicates the risk of the disease and therefore, it was not required to test the system for higher BDNF concentrations. Thesting MQDs-Ab2-d with BSA did not show non-specific binding indicates the specificity of MQDs-Ab2-d with BDNF. Figure 6(c) shows images of control and test reaction tubes after 20 min incubation. The binding reaction of MQDs-Ab2-d and BDNF protein was relatively rapid compared to other biomarkers tested. The binding of MQDs-Ab2-d with BDNF was linear with tested range concentrations (0~10 ng mL-1 BDNF) indicating its broad sensitivity and specificity to BDNF protein in serum.

Multiplexed detection by the developed in vitro PoC immuno-optomagnetic assay potentially ensures the quality, accuracy, and performance of in vitro diagnostics, which will pave the way for novel health monitoring at homes and add value to personalized medicine. Finally, the success and outcomes from this invention will address the current challenges in the field of clinical diagnosis, allow continuously monitoring the disease in the elderly patients, help improving elderly/aged health, provide opportunity for the early detection of disease and prevention, enhance healthy lifespan, compete with existing expensive biomedical practices and opportunity to enter a new global in vitro diagnostic market and improve overall socio-economic development.

### REFERENCES CITED IN THE DESCRIPTION

This list of references cited by the applicant is for the reader's convenience only. It does not form part of the patent document. Even though great care has been taken in compiling the references, errors or omissions cannot be excluded in this regard.

### Non-patent literature cited in the description

[1] M. Paraskevaidi, P.L. Martin-Hirsch, F.L. Martin, Progress and Challenges in the Diagnosis of Dementia: A Critical Review, ACS Chemical Neuroscience (2018).
[2] J. Georges, Estimating the prevalence of dementia in Europe, 2019. https://www.alzheimer-europe.org/content/download/195515/1457520/file/FINAL%2005707%20Alzheimer%20 Europe%20vearbook%202019.pdf. (Accessed 04 Oct. 2021.
[3] D. Arslantas, D. Ozbabalik, S. Metintas, S. Ozkan, C. Kalyoncu, G. Ozdemir, A. Arslantas, Prevalence of dementia and associated risk factors in Middle Anatolia, Turkey, J Clin Neurosci 16(11) (2009) 1455-9.
[4] S. Uslu, Z.E. Akarkarasu, D. Ozbabalik, S. Ozkan, O. Çolak, E.S. Demirkan, A. Ozkiris, C. Demirustu, O. Alatas, Levels of amyloid beta-42, interleukin-6 and tumor necrosis factor-alpha in Alzheimer's disease and vascular dementia, Neurochemical research 37(7) (2012) 1554-1559.
[5] Türkiye-Alze 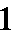 mer-derne 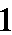 , Geli 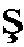 mi 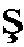 ülkelerde Alzheimer hastal 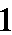 g 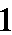 azalirken, Türkiye'de art 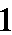 yor!, 2014. (Accessed 26 Feb 2018 2018).
[6] Y. Üresin, H. İlbars, İ.H. Gürvit, M. Emre, Chapter 17 - Experience in Alzheimer's Disease Clinical Trials in Turkey A2 - Bairu, Menghis, in: M.W. Weiner (Ed.), Global Clinical Trials for Alzheimer's Disease, Academic Press, San Diego, 2014, pp. 277-288.
[7] J.A. Schneider, Z. Arvanitakis, W. Bang, D.A. Bennett, Mixed brain pathologies account for most dementia cases in community-dwelling older persons, Neurology 69(24) (2007) 2197-2204.
[8] R. Craig-Schapiro, M. Kuhn, C. Xiong, E.H. Pickering, J. Liu, T.P. Misko, R.J. Perrin, K.R. Bales, H. Soares, A.M. Fagan, D.M. Holtzman, Multiplexed immunoassay panel identifies novel CSF biomarkers for Alzheimer's disease diagnosis and prognosis, PLoS One 6(4) (2011) e18850.
[9] B. Dubois, H.H. Feldman, C. Jacova, S.T. Dekosky, P. Barberger-Gateau, J. Cummings, A. Delacourte, D. Galasko, S. Gauthier, G. Jicha, K. Meguro, J. O'Brien, F. Pasquier, P. Robert, M. Rossor, S. Salloway, Y. Stern, P.J. Visser, P. Scheltens, Research criteria for the diagnosis of Alzheimer's disease: revising the NINCDS-ADRDA criteria, Lancet Neurol 6(8) (2007) 734-46.
[10] M. Ewers, R.A. Sperling, W.E. Klunk, M.W. Weiner, H. Hampel, Neuroimaging markers for the prediction and early diagnosis of Alzheimer's disease dementia, Trends Neurosci 34(8) (2011) 430-442.
[11] K.B. Shanthi, S. Krishnan, P. Rani, A systematic review and meta-analysis of plasma amyloid 1-42 and tau as biomarkers for Alzheimer's disease, SAGE Open Med 3 (2015) 2050312115598250.
[12] O. Tokel, F. Inci, U. Demirci, Advances in Plasmonic Technologies for Point of Care Applications, Chemical Reviews 114(11) (2014) 5728-5752.
[13] J. Singh, M.S. Akbar, S. Adabag, Discordance of cardiac troponin I assays on the point-of-care i-STAT and Architect assays from Abbott Diagnostics, Clinica Chimica Acta 403(1-2) (2009) 259-260.
[14] C. Dincer, R. Bruch, A. Kling, P.S. Dittrich, G.A. Urban, Multiplexed Point-of-Care Testing - xPOCT, Trends Biotechnol 35(8) (2017) 728-742.
[15] A. Polacchini, G. Metelli, R. Francavilla, G. Baj, M. Florean, L.G. Mascaretti, E. Tongiorgi, A method for reproducible measurements of serum BDNF: comparison of the performance of six commercial assays, Sci Rep 5 (2015) 17989.
[16] W.K. Bae, K. Char, H. Hur, S. Lee, Single-step synthesis of quantum dots with chemical composition gradients, Chem Mater 20(2) (2008) 531-539.
[17] Y. Wang, V.D. Ta, Y. Gao, T.C. He, R. Chen, E. Mutlugun, H.V. Demir, H.D. Sun, Stimulated emission and lasing from CdSe/CdS/ZnS core-multi-shell quantum dots by simultaneous three-photon absorption, Adv Mater 26(18) (2014) 2954-61.
[18] Y.J. Zhang, A.M. Schnoes, A.R. Clapp, Dithiocarbamates as Capping Ligands for Water-Soluble Quantum Dots, Acs Appl Mater Inter 2(11) (2010) 3384-3395.
[19] F. Dubois, B. Mahler, B. Dubertret, E. Doris, C. Mioskowski, A versatile strategy for quantum dot ligand exchange, J Am Chem Soc 129(3) (2007) 482-483.
[20] V. Panwar, P. Kumar, A. Bansal, S.S. Ray, S.L. Jain, PEGylated magnetic nanoparticles (PEG@Fe3O4) as cost effective alternative for oxidative cyanation of tertiary amines via CH activation, Applied Catalysis A: General 498 (2015) 25-31.
[21] M. Schmidt, C. Sachse, W. Richter, C. Xu, M. Fandrich, N. Grigorieff, Comparison of Alzheimer Abeta(1-40) and Abeta(1-42) amyloid fibrils reveals similar protofilament structures, Proc Natl Acad Sci U S A 106(47) (2009) 19813-8.
[22] M. Schmidt, A. Rohou, K. Lasker, J.K. Yadav, C. Schiene-Fischer, M. Fandrich, N. Grigorieff, Peptide dimer structure in an Abeta(1-42) fibril visualized with cryo-EM, Proc Natl Acad Sci U S A 112(38) (2015) 11858-63.
[23] J.C. Park, S.H. Han, D. Yi, M.S. Byun, J.H. Lee, S. Jang, K. Ko, S.Y. Jeon, Y.S. Lee, Y.K. Kim, D.Y. Lee, I. Mook-Jung, Plasma tau/amyloid-betal-42 ratio predicts brain tau deposition and neurodegeneration in Alzheimer's disease, Brain 142(3) (2019) 771-786.
[24] R. Christensen, A.B. Marcussen, G. Wortwein, G.M. Knudsen, S. Aznar, Abeta(1-42) injection causes memory impairment, lowered cortical and serum BDNF levels, and decreased hippocampal 5-HT(2A) levels, Exp Neurol 210(1) (2008) 164-71.

## Claims

1. A method for detecting and quantifying of Aβ1-40, Aβ1-42, tau-protein or BDNF as a target analyte in a test sample for Alzheimer's disease which is a central nervous system disease, comprising the steps of:
• Providing free/colloidal bioactivated CdSe/CdS/ZnS non-magnetic quantum dot (QD) of one color functionalised with a primary analyte specific reagent,
• Providing a plurality of optomagnetic magnetic quantum dots (MQD) of different color functionalised with a second analyte specific reagent, capable of binding to analyte followed by the primary analyte specific reagent,
• Providing both primary analyte specific reagent and second analyte specific reagent capable of binding to a common target analyte from different sides,
• Contacting said non-magnetic quantum dot (QD) with a target analyte and forming QD nanocrystals-primary analyte specific reagent-target analyte complex which is Complex 1,
• Forming a sandwich Complex 2 between said Complex 1 and magnetic quantum dots (MQD) functionalised with the second analyte specific reagent,
• Magnetic separation of Complex-2 from non-magnetic unbound QDs,
• Visible detection of diagnostic signal originates from the color change after binding of a target analyte with two different colored (Wavelengths) of biofunctionalized MQDs (λ1) and bioactivated QDs (λ2) by a naked eye in real-time after exposing the test sample to a UV-torch.

2. The method according to claim 1, wherein test sample is biological fluids; such as serum, blood, urine, saliva, sweat, CSF or interstitial fluid.

3. The method according to claim 1, wherein analyte specific reagent is antibody or DNA/RNA probe.

4. The method according to claim 1, wherein primary analyte specific reagent is a primary antibody and second analyte specific reagent is a second antibody.

## Patentansprüche

1. Verfahren zum Nachweis und zur Quantifizierung von Aβ1-40, Aß1-42, Tau-Protein oder BDNF als Zielanalyt in einer Testprobe für Alzheimer-Krankheit, die eine Erkrankung des zentralen Nervensystems ist, umfassend die Schritte:
• Bereitstellen von freiem/kolloidalem bioaktiviertem CdSe/CdS/ZnS nichtmagnetischer Quantenpunkt (QD) einer Farbe, funktionalisiert mit einem primären Analyt-spezifischen Reagenz,
• Bereitstellen einer Vielzahl von optomagnetischen magnetischen Quatumpunkten (MQD) unterschiedlicher Farbe, die mit einem zweiten Analyt-spezifischen Reagenz funktionalisiert sind, das an den Analyten binden kann, gefolgt von dem primären Analyt-spezifischen Reagenz,
• Bereitstellen sowohl des primären Analyt-spezifischen Reagenzes als auch des zweiten Analyt-spezifischen Reagenzes, das in der Lage ist, von verschiedenen Seiten an einen gemeinsamen Zielanalyten zu binden,
• Kontaktieren des nichtmagnetischen Quantenpunkts (QD) mit einem Zielanalyten und Bilden von QD-Nanokristallen - primärer Analyt-spezifischer Reagenz-Zielanalyt-Komplex, der Komplex 1 ist,
• Bilden eines Sandwiches Komplex 2 zwischen dem Komplex 1 und magnetischen Quatum Dots (MQD), die mit dem zweiten Analyt-spezifischen Reagenz funktionalisiert sind,
• Magnetische Trennung von Komplex-2 von nichtmagnetischen ungebundenen QDs,
• Der sichtbare Nachweis eines diagnostischen Signals stammt von der Farbänderung nach Bindung eines Zielanalyten mit zwei unterschiedlich gefärbten (Wellenlängen) von biofunktionalisierten MQDs (λ1) und bioaktivierten QDs (λ2) mit bloßem Auge in Echtzeit, nachdem die Testprobe einer UV-Taschenlampe ausgesetzt wurde.

2. Verfahren nach Anspruch 1, wobei die Testprobe biologische Flüssigkeiten wie Serum, Blut, Urin, Speichel, Schweiß, CSF oder interstitielle Flüssigkeit ist.

3. Verfahren nach Anspruch 1, wobei das Analyt-spezifische Reagenz ein Antikörper oder eine DNA/RNA-Sonde ist.

4. Verfahren nach Anspruch 1, wobei das primäre Analyt-spezifische Reagenz ein primärer Antikörper ist und das zweite Analyt-spezifische Reagenz ein zweiter Antikörper ist.

## Revendications

1. Procédé de détection et de quantification de l'Aβ1-40, de l'Aβ1-42, la protéine tau ou le BDNF en tant qu'analyte cible dans un échantillon d'essai pour la maladie d'Alzheimer qui est une maladie du système nerveux central, comprenant les étapes suivantes :
• Fournir un point quantique (QD) non magnétique CdSe/CdS/ZnS bioactivé libre/colloïdal d'une seule couleur fonctionnalisé avec un premier réactif spécifique d'analyte,
• Fournir une pluralité de points quantiques magnétiques optomagnétiques (MQD) de différentes couleurs fonctionnalisés avec un deuxième réactif spécifique d'analyte, capable de se lier à l'analyte suivi du premier réactif spécifique d'analyte.
• Fournir à la fois un premier réactif spécifique d'analyte et un deuxième réactif spécifique d'analyte capables de se lier à un analyte cible commun à partir de côtés différents,
• Mettre en contact ledit point quantique (QD) non magnétique avec un analyte cible et former un complexe QD nanocristaux-premier réactif spécifique d'analyte -analyte cible qui est le Complexe 1,
• Former un complexe sandwich 2 entre ledit complexe 1 et des points quantiques magnétiques (MQD) fonctionnalisés avec le deuxième réactif spécifique d'analyte,
• Séparer magnétiquement le Complexe-2 des QDs non magnétiques non liés,
• Détecter visiblement le signal diagnostique provient du changement de couleur après la liaison d'un analyte cible avec deux couleurs différentes (longueurs d'onde) de MQD biofonctionnalisés (λ1) et de QD bioactivés (λ2) à l'œil nu en temps réel après avoir exposé l'échantillon d'essai à une torche UV.

2. Procédé selon la revendication 1, dans lequel l'échantillon d'essai est un liquide biologique; tel que le sérum, le sang, l'urine, la salive, la sueur, le LCR ou le liquide interstitiel.

3. Procédé selon la revendication 1, dans lequel le réactif spécifique d'analyte est un anticorps ou une sonde ADN/ARN.

4. Procédé selon la revendication 1, dans lequel le premier réactif spécifique d'analyte est un premier anticorps et le deuxième réactif spécifique d'analyte est un deuxième anticorps.
